# EUROPEAN PATENT SPECIFICATION

(11) **EP 4 313 268 B1**
(45) Date of publication and mention of the grant of the patent: **01.04.2026**
(21) Application number: 22738798.2
(22) Date of filing: 06.06.2022
(51) Int. Cl.: A61N 1/36, A61N 1/372

(54) **SYSTEMS FOR CHARGE BALANCING OF ELECTRICAL STIMULATION**
SYSTEME ZUM LADUNGSAUSGLEICH EINER ELEKTRISCHEN STIMULATION
SYSTÈMES D'ÉQUILIBRAGE DE CHARGE DE STIMULATION ÉLECTRIQUE

(30) Priority: 07.06.2021 US 202163197812 P
(43) Date of publication of application: 07.02.2024
(73) Proprietor: Boston Scientific Neuromodulation Corporation, Valencia, CA 91355 (US)
(72) Inventor: FELDEMAN, Emanuel, Simi Valley, CA 93063 (US); KASHYAP, Dheerendra, Raghavendra, Valencia, CA 91354 (US); WEERAKOON, Pujitha, Valencia, CA 91354 (US); WEISS, Philip, Leonard, Sherman Oaks, CA 91401 (US); GRANDHE, Sarvani, Valencia, CA 91354 (US)
(74) Representative: Vossius & Partner Patentanwälte Rechtsanwälte mbB
(86) International application number: PCT/US2022/032357
(87) International publication number: WO 2022/261004

(56) References cited:
- US-A1- 2010 114 261
- US-A1- 2011 046 432
- US-A1- 2011 106 214
- US-A1- 2016 220 820

## Description

### FIELD

The present disclosure is directed to the area of implantable electrical stimulation systems and methods of making and using the systems. The present disclosure is also directed to methods and systems for charge balancing of electrical stimulation.

### BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable electrical stimulation systems have proven therapeutic in a variety of diseases and disorders. For example, spinal cord stimulation systems have been used as a therapeutic modality for the treatment of chronic pain syndromes. Peripheral nerve stimulation has been used to treat chronic pain syndrome and incontinence, with a number of other applications under investigation. Deep brain stimulation can be used to treat a variety of diseases and disorders.

An implantable neurostimulation system may include an implantable neurostimulator, also referred to as an implantable pulse generator (IPG) or other control module, and one or more implantable leads each including one or more electrodes. The implantable neurostimulator delivers neurostimulation energy through one or more electrodes placed on or near a target site in the nervous system. An external programming device is used to program the implantable neurostimulator with stimulation parameters controlling the delivery of the neurostimulation energy.

U.S. Patent Application Publication No. 2016/0220820 discloses a neurostimulation system that includes a storage device to store a stimulation waveform, a programming control circuit to generate a plurality of stimulation parameters controlling delivery of neurostimulation pulses according to the stimulation waveform, and waveform definition circuit configured to create and adjust the stimulation waveform. The waveform definition circuit includes a charge recovery module that may include a stimulation to receive the stimulation waveform including charge injection phases, a charge recovery scheme input to receive a charge recovery scheme, and a waveform adjuster configured to identify a need for recovering charges injected during the charge injection phases and adjust the received stimulation waveform by automatically inserting charge recovery phases into the received stimulation waveform based on the identified need for recovering the injected charges and the received charge recovery scheme.

### BRIEF SUMMARY

One aspect is an electrical stimulation system that includes at least one electrical stimulation lead, each of the at least one electrical stimulation lead including a plurality of stimulation electrodes; and a processor coupled to the at least one electrical stimulation lead, wherein the processor executes instructions that perform actions, including: directing delivery of at least one stimulation pulse through at least one of the stimulation electrodes of the at least one electrical stimulation lead to tissue of a patient during each of a plurality of charge injection phases, wherein each consecutive pair of the charge injection phases is separated by a charge recovery phase; and for at least one of the at least one stimulation pulse: during delivery of the stimulation pulse, directing application of at least one charge recovery pulse to interrupt the delivery of the stimulation pulse, wherein each one of the at least one charge recovery pulse has a relative amplitude that is larger in magnitude than an amplitude of the stimulation pulse; and, for each of the at least one charge recovery pulse, after application of the charge recovery pulse, directing resumption of delivery of the stimulation pulse at the amplitude of the stimulation pulse.

In at least some aspects, applying the at least one charge recovery pulses includes applying a plurality of charge recovery pulses. In at least some aspects, applying the plurality of charge recovery pulses includes applying the plurality of charge recovery pulses periodically with a predetermined period. In at least some aspects, applying the plurality of charge recovery pulses includes successively applying each of the plurality of charge recovery pulses as an unrecovered charged delivered by the at least one stimulation pulse reaches or exceeds a predetermined threshold amount.

In at least some aspects, the at least one charge recovery pulse recovers at least 10% of charge delivered by the at least one stimulation pulse of the at least one of the charge injection phases. In at least some aspects, the actions further include, during the charge recovery phase, provide at least one active recovery phase. In at least some aspects, the actions further include, during the charge recovery phase, provide at least one passive recovery phase.

In at least some aspects, the electrical stimulation system further includes a programming unit configured to communicate with the processor to program the processor. In at least some aspects, the programming unit is configured to receive user input or modification of a duration of the at least one charge recovery pulse and to communicate the user input to the processor. In at least some aspects, the programming unit is configured to receive user input or modification of an amplitude or a relative amplitude of the at least one charge recovery pulse and to communicate the user input to the processor.

Another aspect is an electrical stimulation system that includes at least one electrical stimulation lead, each of the at least one electrical stimulation lead including a plurality of stimulation electrodes; and a processor coupled to the at least one electrical stimulation lead, wherein the processor executes instructions that perform actions, including: directing delivery of at least one stimulation pulse through at least one of the stimulation electrodes of the at least one electrical stimulation lead to tissue of a patient during each of a plurality of charge injection phases, wherein each consecutive pair of the charge injection phases is separated by a charge recovery phase; and during at least one of the charge injection phases, applying at least one charge recovery pulse to interrupt the delivery of the at least one stimulation pulse of the at least one of the charge injection phases, wherein each of the at least one charge recovery pulse has a duration of no more than 20 microseconds.

In at least some aspects, the directing includes directing delivery of a one of the at least one stimulation pulse immediately prior to, and immediately after, the application of a one of the least one charge recovery pulse. In at least some aspects, the applying includes interrupting the delivery of a one of the least one stimulation pulse by the application of a one of the at least one charge recovery pulse, and wherein the directing delivery includes resuming the one of the at least one stimulation pulse following the application of the one of the at least one charge recovery pulse.

In at least some aspects, the at least one charge recovery pulse recovers at least 10% of charge delivered by the at least one stimulation pulse of the at least one of the charge injection phases. In at least some aspects, the actions further include, during the charge recovery phase, provide at least one active recovery phase. In at least some aspects, the actions further include, during the charge recovery phase, provide at least one passive recovery phase. In at least some aspects, the electrical stimulation system further includes a programming unit configured to communicate with the processor to program the processor.

A further aspect is a method of delivering electrical stimulation that includes delivering at least one stimulation pulse through at least one stimulation electrode of at least one electrical stimulation lead to tissue of a patient during each of a plurality of charge injection phases, wherein each consecutive pair of the charge injection phases is separated by a charge recovery phase; and during at least one of the charge injection phases, applying at least one charge recovery pulse to interrupt delivery of the at least one stimulation pulse of the at least one of the charge injection phases, wherein each of the at least one charge recovery pulse has a duration of no more than 20 microseconds.

In at least some aspects, the delivering includes delivering a one of the at least one stimulation pulse immediately prior to, and immediately after, the application of a one of the least one charge recovery pulse. In at least some aspects, the applying includes interrupting the delivery of a one of the least one stimulation pulse by application of a one of the at least one charge recovery pulse, and wherein the directing delivery includes resuming the one of the at least one stimulation pulse following the application of the one of the at least one charge recovery pulse.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system;
FIG. 2 is a schematic illustration of one example of stimulation pulses with a passive recovery phase and an active recovery phase;
FIG. 3A is a schematic illustration of another example of stimulation pulses delivered during charge injection phases separated by a charge recovery phase;
FIG. 3B is a schematic illustration of the example of FIG. 3A with the application of multiple charge recovery pulses during the charge injection phases;
FIG. 4 is a schematic overview of one embodiment of components of a stimulation system, including an electronic subassembly disposed within a control module;
FIG. 5 is a schematic block diagram of elements of an electrical stimulation system;
FIG. 6 is a schematic diagram of a user interface for programming parameters of charge recovery pulses; and
FIG. 7 is a flowchart of one method of delivering electrical stimulation using charge recovery pulses.

### DETAILED DESCRIPTION

The present disclosure is directed to the area of implantable electrical stimulation systems and methods of making and using the systems. The present disclosure is also directed to methods and systems for charge balancing of electrical stimulation.

Suitable implantable electrical stimulation systems or other neurostimulation systems can include, but are not limited to, a least one lead with one or more electrodes disposed on a distal portion of the lead and one or more terminals disposed on one or more proximal portions of the lead. Leads include, for example, percutaneous leads, paddle leads, cuff leads, or any other arrangement of electrodes on a lead. Examples of electrical stimulation systems with leads are found in, for example, U.S. Patents Nos. 6,181,969; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,175,710; 8,224,450; 8,271,094; 8,295,944; 8,364,278; 8,391,985; and 8,688,235; and U.S. Patent Applications Publication Nos. 2007/0150036; 2009/0187222; 2009/0276021; 2010/0076535; 2010/0268298; 2011/0005069; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; 2013/0105071; and 2013/0197602.

In the discussion below, a percutaneous lead will be exemplified, but it will be understood that the methods and systems described herein are also applicable to paddle leads and other leads.

A percutaneous lead for electrical stimulation (for example, deep brain, spinal cord, or peripheral nerve stimulation) includes stimulation electrodes that can be ring electrodes, segmented electrodes that extend only partially around the circumference of the lead, or any other type of electrode, or any combination thereof. The segmented electrodes can be provided in sets of electrodes, with each set having electrodes circumferentially distributed about the lead at a particular longitudinal position. A set of segmented electrodes can include any suitable number of electrodes including, for example, two, three, four, or more electrodes. In at least some embodiments, the leads are used for spinal cord stimulation, deep brain stimulation, peripheral nerve stimulation, dorsal root ganglion stimulation, sacral nerve stimulation, or stimulation of other nerves, muscles, and tissues, or for any other suitable type of stimulation.

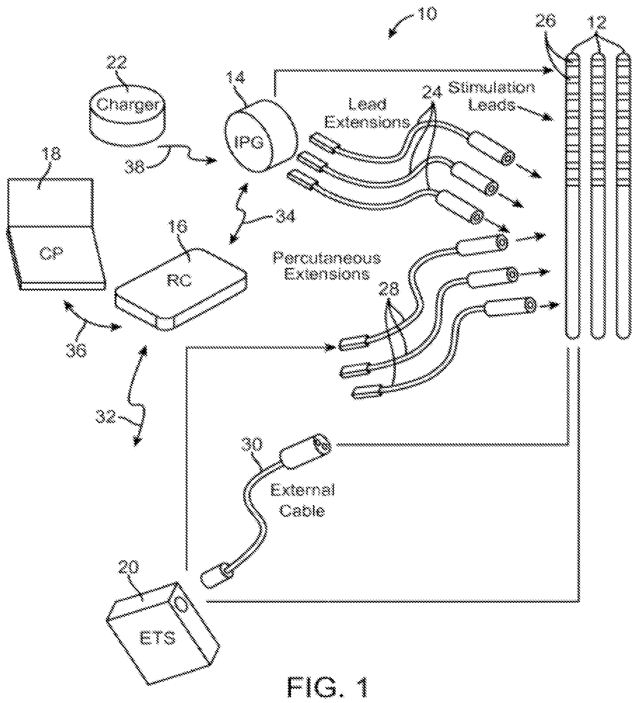

Turning to Figure 1, one embodiment of an electrical stimulation system 10 includes one or more stimulation leads 12 and an implantable pulse generator (IPG) 14. The system 10 can also include one or more of an external remote control (RC) 16, a clinician's programmer (CP) 18, an external trial stimulator (ETS) 20, or an external charger 22. The IPG and ETS are examples of control modules for the electrical stimulation system.

The IPG 14 is physically connected, optionally via one or more lead extensions 24, to the stimulation lead(s) 12. Each lead carries multiple electrodes 26 arranged in an array. The IPG 14 includes pulse generation circuitry that delivers electrical stimulation energy in the form of, for example, a pulsed electrical waveform (i.e., a temporal series of electrical pulses) to the electrode array 26 in accordance with a set of stimulation parameters. The implantable pulse generator can be implanted into a patient's body, for example, below the patient's clavicle area or within the patient's buttocks or abdominal cavity or at any other suitable site. The implantable pulse generator can have multiple stimulation channels which may be independently programmable to control the magnitude of the current stimulus from each channel. In some embodiments, the implantable pulse generator can have any suitable number of stimulation channels including, but not limited to, 4, 6, 8, 12, 16, 32, or more stimulation channels. The implantable pulse generator can have one, two, three, four, or more connector ports, for receiving the terminals of the leads and/or lead extensions.

The ETS 20 may also be physically connected, optionally via the percutaneous lead extensions 28 and external cable 30, to the stimulation leads 12. The ETS 20, which may have similar pulse generation circuitry as the IPG 14, also delivers electrical stimulation energy in the form of, for example, a pulsed electrical waveform to the electrode array 26 in accordance with a set of stimulation parameters. One difference between the ETS 20 and the IPG 14 is that the ETS 20 is often a non-implantable device that is used on a trial basis after the neurostimulation leads 12 have been implanted and prior to implantation of the IPG 14, to test the responsiveness of the stimulation that is to be provided. Any functions described herein with respect to the IPG 14 can likewise be performed with respect to the ETS 20.

The RC 16 may be used to telemetrically communicate with or control the IPG 14 or ETS 20 via a uni- or bi-directional wireless communications link 36, 32. Once the IPG 14 and neurostimulation leads 12 are implanted, the RC 16 may be used to telemetrically communicate with or control the IPG 14 via a uni- or bi-directional communications link 34. Such communication or control allows the IPG 14 to be turned on or off and to be programmed with different stimulation parameter sets. The IPG 14 may also be operated to modify the programmed stimulation parameters to actively control the characteristics of the electrical stimulation energy output by the IPG 14. The CP 18 allows a user, such as a clinician, the ability to program stimulation parameters for the IPG 14 and ETS 20 in the operating room and in follow-up sessions. Alternately, or additionally, stimulation parameters can be programed via wireless communications (e.g., Bluetooth) between the RC 16 (or external device such as a hand-held electronic device) and the IPG 14.

The CP 18 may perform this function by indirectly communicating with the IPG 14 or ETS 20, through the RC 16, via a wireless communications link 36, 32. Alternatively, the CP 18 may directly communicate with the IPG 14 or ETS 20 via a wireless communications link (not shown). The stimulation parameters provided by the CP 18 are also used to program the RC 16, so that the stimulation parameters can be subsequently modified by operation of the RC 16 in a stand-alone mode (i.e., without the assistance of the CP 18) through a communications link 34. The external charger 22 can provide power to the IPG 14 through a link 38.

Examples of electrical stimulation systems can be found at U.S. Patents Nos. 6,181,969; 6,516,227; 6,609,029; 6,609,032; 6,741,892; 6,895,280; 7,949,395; 7,244,150; 7,672,734; and 7,761,165; 7,974,706; 8,175,710; 8,224,450; and 8,364,278; and U.S. Patent Application Publication No. 2007/0150036.

In at least some embodiments, the neurostimulation energy is delivered in the form of electrical stimulation pulses. A stimulation pulse (or set of stimulation pulses) may include a charge injection phase followed by a charge recovery phase for patient safety or longevity of electrodes used to deliver the pulses. During the charge injection phase, charge is injected into targeted neural tissue using electrodes including at least one anode and at least one cathode. In at least some embodiments, a case of the IPG may act as one of the electrodes. In at least some embodiments, during the charge recovery phase, the anode and cathode are switched to recover the injected charge. Without recovering the injected charge, the neural tissue maybe harmed or the electrodes may suffer long term damage. Examples of patterns of charge injection and recovery phases are presented in U.S Patent Application Publications Nos. 2014/0243924 and 2018/0071527 and U.S. Patent No. 10,335,599.

As an example, electrical stimulation can include one or more neural stimulation pulse delivered during one or more charge injection (or stimulation) phases. A charge injection phase can be followed a charge recovery phase which can include one or more active, or passive, charge recovery phases. An active recovery phase may include applying a pulse that is opposite in phase to the pulses of the charge injection phase(s). Passive recovery can be implemented using, for example, passive recovery switches (for example, transistors) that are connected between electrode nodes and a common reference voltage. In at least some embodiments, closing the passive recovery switches couples DC-blocking capacitors in parallel between the reference voltage and the patient's tissue to recover charge. Examples of passive recovery phases are presented in U.S. Patent Application Publication No. 2018/0071527 and U.S. Patent No. 10,335,599.

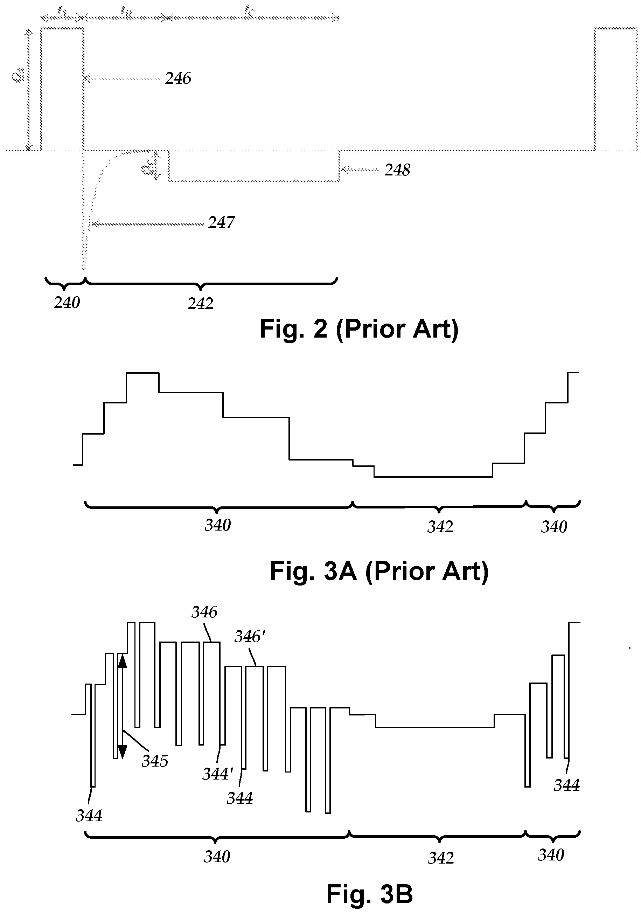

Figure 2 illustrates a charge injection phase 240 with a stimulation pulse 246 where a charge Qs is injected during a time ts. In Figure 2, the charge recovery phase 242 includes a passive recovery phase 247 and an active recovery phase 248. (Other charge recovery phases may include only an active recovery phase or a passive recovery phase or any suitable combination of active and passive recovery phases.) After a delay time t_{D}, the active recovery phase 248 recovers a charge Q_{C} over time tc. In at least some embodiments, Q_{S} = Q_{C} or Q_{S} > Qc. Additional charge recovery occurs with the passive recovery phase 247. This cycle may repeat over periodic time, t_{P}. In at least some embodiments, in a charge-balanced stimulation, at least part of, the charge injected into the tissue during the stimulation phase is recovered for neuron safety or to limit stimulation capacitor voltage buildup to meet the stimulation current DACs (Digital-to-Analog Converters) compliance voltage requirements.

In a simple, tonic train of identical neurostimulation pulses, a charge recovery phase may follow a charge injection pulse for each of the neurostimulation pulses. This may not be possible for a complex pattern of neurostimulation pulses. For example, following the charge injection phase of a neurostimulation pulse, there may not be sufficient time for completely recovering the injected charge before the charge injection phase of the next neurostimulation pulse is programmed to start. Thus, for a rapid burst of neurostimulation pulses, the charge injection phases may occur in sequence followed by one or more charge recovery phases to provide or increase charge balance.

In addition, for long charge injection phase times, the charge recovery phase may be delayed to a point where there may be a safety issue. This may become a more pronounced concern when using complex or arbitrary stimulation waveforms, which may be long or oddly shaped. Neural sensing may also add delay to charge recovery as charge recovery may not be available or possible during the sensing period for the neural response. Examples of other periods of time when charge balance may also be an issue: a) during stimulation ramp-up; b) when changing stimulation programs, or c) during stimulation with high pulse rates or wide pulses when charge recovery time may not be sufficient.

Conventional charge recovery methods can include setting limits on the maximum pulse width and providing a minimum recovery duration for the active or passive recovery phases to limit the maximum charge and the charge density per charge injection phase. Other charge recovery methods include using a static recovery mode in which the lead provides a constant mechanism for recovering charge.

In contrast to these conventional methods and as described herein, active recovery can be implemented during the charge injection phase(s). In at least some embodiments, to provide for at least some portion of the charge recovery, charge recovery pulses can be included during the charge injection phase(s). In at least some embodiments, the charge recovery pulses are relatively short, for example, as compared to the length of the charge injection phase(s) or stimulation pulses.

Figure 3A illustrates one example of a conventional arrangement with charge injection phases 340 (having one or more stimulation pulses 346) and a charge recovery phase 342 (optionally having one or more passive recovery phases or active recovery phases or any combination thereof) between the charge injection phases. Figure 3B illustrates the same charge injection phases 340 and charge recovery phase 342 with the addition of multiple charge recovery pulses 344 occurring during the charge injection phases. In at least some embodiments, each charge recovery pulse 344 is opposite in phase to the stimulation pulses 346. In at least some embodiments, the charge recovery pulse 344 can be considered to interrupt a stimulation pulse 346 with the stimulation pulse 346 resuming after the charge recovery pulse.

The term "relative amplitude" 345 (illustrated in Figure 3B) is the difference between the amplitude, relative to a reference level (e.g., zero voltage or zero current) of the stimulation pulse 346 which the charge recovery pulse 344 interrupts and the amplitude, relative to the reference level, of the charge recovery pulse. The term "amplitude" without the modifier "reference" is used to indicate the amplitude relative to the reference level (e.g., zero voltage or zero current). In at least some embodiments, the relative amplitude of the charge recovery pulse 344 is larger in magnitude than the amplitude of the stimulation pulse 346 that the charge recovery pulse interrupts. In at least some embodiments, a charge recovery pulse 344' may bridge two stimulation pulses 346, 346', as illustrated in Figure 3B.

In at least some embodiments, the charge recovery pulses 344 are utilized in conjunction with one or more charge recovery phases that can include one or more active recovery phase(s), passive recovery phase(s), or static charge recovery or any combination thereof. In at least some embodiments, charge recovery is provided solely using charge recovery pulses 344 during the charge injection phases 340. In at least some embodiments, the charge recovery pulses 344 recover at least 10, 20, 25, 33, 50, 75, 80, 90, 95, 99, or 100 percent of the charge introduced during the charge injection phase(s) 342. In at least some embodiments, a remainder of the charge introduced during the charge injection phase(s) 342 is recovered using one or more of active recovery phase(s), passive recovery phase(s), or static charge recovery or any combination thereof.

The charge recovery pulses 344 have a relatively short duration. In at least some embodiments, the charge recovery pulse 344 has a duration of no more than 30, 20, 10, 5, or 1 microseconds. In at least some embodiments, the duration of the charge recovery pulse 344 (or the total duration of the charge recovery pulses) is no more than 50, 30, 25, 20, 10, 5, 2, or 1% of the duration of the charge injection phase during which the charge recovery pulse(s) occurs. In at least some embodiments, the charge recovery pulses 344 have the same duration. In at least some embodiments, the charge recovery pulses 344 can have different durations. In at least some embodiments, the duration(s) of the charge recovery pulses 344 can be selected or modified by a user through the IPG, CP, RC, or ETS (or any combination thereof). In at least some embodiments, the IPG, CP, RC, or ETS may have a default duration of the charge recovery pulses 344. In at least some embodiments, the duration(s) of the charge recovery pulses 344 may be selected by (or have a default value in) the IPG, CP, RC, or ETS (or any combination thereof) and are, optionally, user-modifiable.

The charge recovery pulses 344 can have a high relative amplitude (for example, the relative amplitude can be equal to or greater in magnitude than the amplitude of one or more of the charge injection pulse(s) of the charge injection phase) which can result in change in phase of the delivered current or voltage. In at least some embodiments, all of the charge recovery pulses 344 have the same amplitude or the same relative amplitude. In at least some embodiments, the charge recovery pulses 344 can have different amplitudes or different relative amplitudes. In at least some embodiments, the amplitude(s) or relative amplitude(s) of the charge recovery pulses 344 can be selected or modified by a user through the IPG, CP, RC, or ETS (or any combination thereof). In at least some embodiments, the IPG, CP, RC, or ETS may have a default amplitude or relative amplitude of the charge recovery pulses 344. In at least some embodiments, the amplitude(s) or relative amplitude(s) of the charge recovery pulses 344 may be selected by (or have a default value in) the IPG, CP, RC, or ETS (or any combination thereof) and are, optionally, user-modifiable.

In at least some embodiments, the effect of charge recovery pulses 344 is sub-threshold so that the person receiving electrical stimulation does not feel any effect from the charge recovery pulses. In at least some embodiments, even though the amplitude of the charge recovery pulses 344 is relatively large, the effect of the charge recovery pulses remains sub-threshold due, at least in part, to the relatively short duration of the charge recovery pulses. In other embodiments, the person receiving electrical stimulation may feel an effect from the charge recovery pulses 344.

In at least some embodiments, the effect of charge recovery pulses 344 is sub-threshold and the charge recovery pulses do not trigger a neural response. In other embodiments, the charge recovery pulses 344 may or do trigger a neural response.

In at least some embodiments, the charge recovery pulses 344 are applied on a regular, periodic schedule. In at least some embodiments, the period of the schedule may be user-selected. In at least some embodiments, the period of the schedule can be determined by one of the IPG, CP, RC, or ETS or any combination thereof. For example, the IPG, CP, RC, or ETS (or any combination thereof) may determine the period of the schedule based on the estimated charge delivered using the stimulation pulse(s) 346 and the duration(s) and amplitude(s) of the charge recovery pulses. The IPG, CP, RC, or ETS (or any combination thereof) may also select, modify, or adjust the duration(s) and amplitude(s) of the charge recovery pulses. The determination may also account for any active or passive recovery phases during the charge recovery phase(s) 342.

In at least some embodiments, the charge recovery pulses 344 can be applied on an irregular schedule. In at least some embodiments, the irregular schedule can be selected or modified by the user. In at least some embodiments, the irregular schedule can be determined by one of the IPG, CP, RC, or ETS or any combination thereof. For example, the IPG, CP, RC, or ETS (or any combination thereof) may apply a charge recovery pulse 344 based on the estimated charge delivered (optionally, reduced by previous charge recovery) using the stimulation pulse(s) 346 up to that point in time and apply the charge recovery pulse when a threshold amount of unrecovered charge has been delivered or exceeded. In at least some embodiments, the IPG may make the determination to apply a charge recovery pulse 344 during delivery of the stimulation pulse(s) 346 to provide a real-time control for charge recovery. In at least some embodiments, the IPG may make the determination to apply a charge recovery pulse 344 during delivery of the stimulation pulse(s) 346 in response measurements or sensing that provides information about the charge delivered or charge build-up in the tissue, lead, electrodes, or elsewhere.

In at least some embodiments, any combination of regular and irregular schedules may be used for determining the delivery of the charge recovery pulses 344.

In at least some embodiments, the addition of charge recovery pulses 344 to the stimulation pulse(s) 346 can be accomplished by modifying the programming of the stimulation pulse(s) to provide the more complicated stimulation pattern. Examples of programming stimulation pattems are presented in the references cited herein, as well as U.S. Patent Application Publication No. 2016/0001087

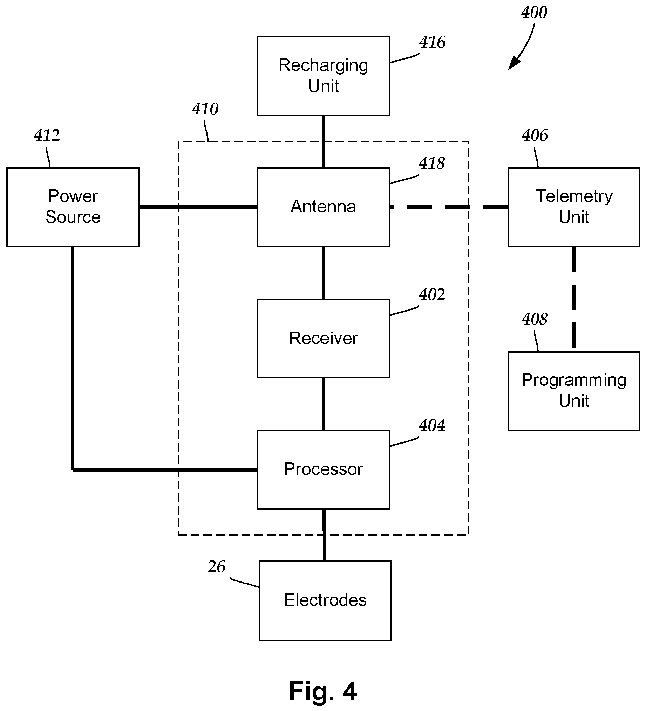

Figure 4 is a schematic overview of one embodiment of components of an electrical stimulation system 400 including an electronic subassembly 410 that is part of the IPG or other control module. It will be understood that the electrical stimulation system can include more, fewer, or different components and can have a variety of different configurations including those configurations disclosed in the stimulator references cited herein.

Some of the components (for example, a power source 412, an antenna 418, a receiver 402, and a processor 404) of the electrical stimulation system can be positioned on one or more circuit boards or similar carriers within a sealed housing of an implantable pulse generator (see e.g., IPG 14 in Figure 1), if desired. Any power source 412 can be used including, for example, a battery such as a primary battery or a rechargeable battery. Examples of other power sources include super capacitors, nuclear or atomic batteries, mechanical resonators, infrared collectors, thermally-powered energy sources, flexural powered energy sources, bioenergy power sources, fuel cells, bioelectric cells, osmotic pressure pumps, and the like including the power sources described in U.S. Patent No. 7,437,193.

As another alternative, power can be supplied by an external power source through inductive coupling via the optional antenna 418 or a secondary antenna. The external power source can be in a device that is mounted on the skin of the user or in a unit that is provided near the user on a permanent or periodic basis.

If the power source 412 is a rechargeable battery, the battery may be recharged using the optional antenna 418, if desired. Power can be provided to the battery for recharging by inductively coupling the battery through the antenna to a recharging unit 416 external to the user. Examples of such arrangements can be found in the references identified above.

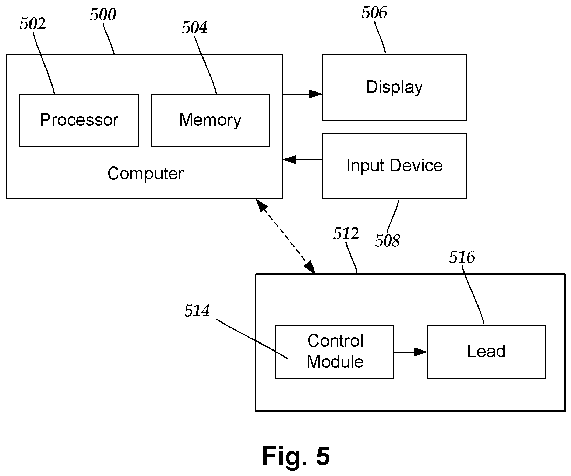

The electronic subassembly 410 and, optionally, the power source 412 can be disposed within a control module (e.g., the IPG 14 or the ETS 20 of Figure 1). The control module is also shown in Figure 5.

In one embodiment, electrical stimulation signals are emitted by the electrodes 26 (Figure 1) on the paddle or lead body to stimulate nerve fibers, muscle fibers, or other body tissues near the electrical stimulation system. The processor 404 is generally included to control the timing and electrical characteristics of the electrical stimulation system. For example, the processor 404 can, if desired, control one or more of the timing, frequency, strength, duration, and waveform of the stimulation pulses, the charge recovery pulses, and any active or passive recovery. In addition, the processor 404 can select which electrodes can be used to provide stimulation, if desired. In some embodiments, the processor 404 selects which electrode(s) are cathodes and which electrode(s) are anodes. In some embodiments, the processor 404 is used to identify which electrodes provide the most useful stimulation of the desired tissue.

Any processor can be used and can be as simple as an electronic device that, for example, produces pulses at a regular interval or the processor can be capable of receiving and interpreting instructions from an external programming unit 408 that, for example, allows modification of pulse characteristics. In the illustrated embodiment, the processor 404 is coupled to a receiver 402 which, in turn, is coupled to the optional antenna 418. This allows the processor 404 to receive instructions from an external source to, for example, direct the pulse characteristics and the selection of electrodes, if desired.

In one embodiment, the antenna 418 is capable of receiving signals (e.g., RF signals) from an external telemetry unit 406 which is programmed by the programming unit 408. The programming unit 408 can be external to, or part of, the telemetry unit 406. For example, the telemetry unit 506 and programming unit 408 can be the RC 16 or CP 18 of Figure 1. The telemetry unit 406 can be a device that is worn on the skin of the user or can be carried by the user and can have a form similar to a pager, cellular phone, or remote control, if desired. As another alternative, the telemetry unit 406 may not be worn or carried by the user but may only be available at a home station or at a clinician's office. The programming unit 408 can be any unit that can provide information to the telemetry unit 406 for transmission to the electrical stimulation system 400. The programming unit 408 can be part of the telemetry unit 406 or can provide signals or information to the telemetry unit 406 via a wireless or wired connection. One example of a suitable programming unit is a computer operated by the user or clinician to send signals to the telemetry unit 406.

The signals sent to the processor 404 via the antenna 418 and the receiver 402 can be used to modify or otherwise direct the operation of the electrical stimulation system. For example, the signals may be used to modify the pulses of the electrical stimulation system such as modifying one or more of pulse duration, pulse frequency, pulse waveform, and pulse strength. The signals may also direct the electrical stimulation system 400 to cease operation, to start operation, to start charging the battery, or to stop charging the battery. In other embodiments, the stimulation system does not include the antenna 418 or receiver 402 and the processor 404 operates as programmed.

Optionally, the electrical stimulation system 400 may include a transmitter (not shown) coupled to the processor 404 and the antenna 418 for transmitting signals back to the telemetry unit 406 or another unit capable of receiving the signals. For example, the electrical stimulation system 400 may transmit signals indicating whether the electrical stimulation system 400 is operating properly or not or indicating when the battery needs to be charged or the level of charge remaining in the battery. The processor 404 may also be capable of transmitting information about the pulse characteristics so that a user or clinician can determine or verify the characteristics.

Figure 5 illustrates one embodiment of a system for practicing the invention. The system can include a computer 500 or any other similar device that includes a processor 502 and a memory 504, a display 506, an input device 508, and, optionally, the electrical stimulation system 512.

The computer 500 can be a laptop computer, desktop computer, tablet, mobile device, smartphone, or other devices that can run applications or programs, or any other suitable device for processing information and for presenting a user interface. The computer can be, for example, the CP 18 or RC 16 of Figure 1. The computer 500 can be local to the user or can include components that are non-local to the user including one or both of the processor 502 or memory 504 (or portions thereof). For example, in some embodiments, the user may operate a terminal that is connected to a non-local computer. In other embodiments, the memory can be non-local to the user. As another example, the computer 500 may utilize or communicate with a processor in the control module 514 (such as the IPG 14 or ETS 20 of Figure 1).

The computer 500 can utilize any suitable processor 502 including one or more hardware processors that may be local to the user or non-local to the user or other components of the computer. The processor 502 is configured to execute instructions provided to the processor, as described below.

Any suitable memory 504 can be used for the processor 502. The memory 504 illustrates a type of computer-readable media, namely computer-readable storage media. Computer-readable storage media may include, but is not limited to, nonvolatile, non-transitory, removable, and non-removable media implemented in any method or technology for storage of information, such as computer readable instructions, data structures, program modules, or other data. Examples of computer-readable storage media include RAM, ROM, EEPROM, flash memory, or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computer.

Communication methods provide another type of computer readable media; namely communication media. Communication media typically embodies computer-readable instructions, data structures, program modules, or other data in a modulated data signal such as a carrier wave, data signal, or other transport mechanism and include any information delivery media. The terms "modulated data signal," and "carrier-wave signal" includes a signal that has one or more of its characteristics set or changed in such a manner as to encode information, instructions, data, and the like, in the signal. By way of example, communication media includes wired media such as twisted pair, coaxial cable, fiber optics, wave guides, and other wired media and wireless media such as acoustic, RF, infrared, and other wireless media.

The display 506 can be any suitable display device, such as a monitor, screen, display, or the like, and can include a printer. The input device 508 can be, for example, a keyboard, mouse, touch screen, track ball, joystick, voice recognition system, or any combination thereof, or the like and can be used by the user to interact with a user interface or clinical effects map.

The electrical stimulation system 512 can include, for example, a control module 514 (for example, the IPG 14 or ETS 20 of Figure 1) and a lead 516 (for example, the lead 12 illustrated in Figure 1.) The electrical stimulation system 512 may communicate with the computer 500 through a wired or wireless connection or, alternatively or additionally, a user can provide information between the electrical stimulation system 512 and the computer 500 using a computer-readable medium or by some other mechanism. In some embodiments, the computer 500 may include part of the electrical stimulation system. In at least some embodiments, the computer 500 can program the control module 514 for delivery of stimulation pulses, charge recovery pulse, charge recovery phases, or the like or any combination thereof.

Electrical stimulation systems can provide a user interface that facilitates parameter selections. Examples of such systems and interfaces can be found in, for example, U.S. Patents Nos. 8,326,433; 8,831,731; 8,849,632; 9,050,470; and 9,072,905; and U.S. Patent Application Publication No. 2014/0277284.

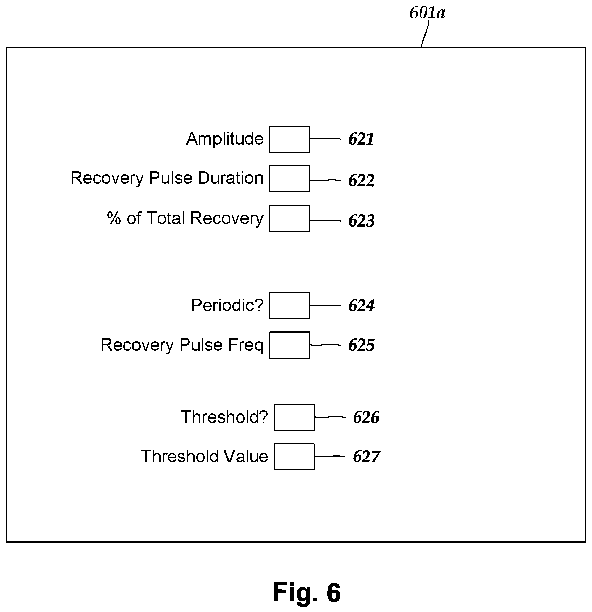

Turning to Figure 6, a user interface of the computer 500 (e.g., the CP 18, RC 16, ETS 20, or IPG 14 of Figure 1) can include user-selectable controls for selecting, adjusting, or modifying parameters related to the charge recovery pulses 344. Figure 6 shows a display 601a with user-selectable controls 621-627 that enable a user to select values for different parameters for the charge recovery pulses 344. Different embodiments of the user interface may have more or fewer user-selectable controls. Examples of the user-selectable controls include, but are not limited to, amplitude 621, recovery pulse duration 622, percent of total recovery to be provided by the charge recovery pulses 623, whether the charge recovery pulses are periodic 624, a pulse frequency 625 of the charge recovery pulses 344 (if periodic), whether the charge recovery pulses are applied when an unrecovered charge injection threshold is met 626, and a value for the unrecovered charge injection threshold 627.

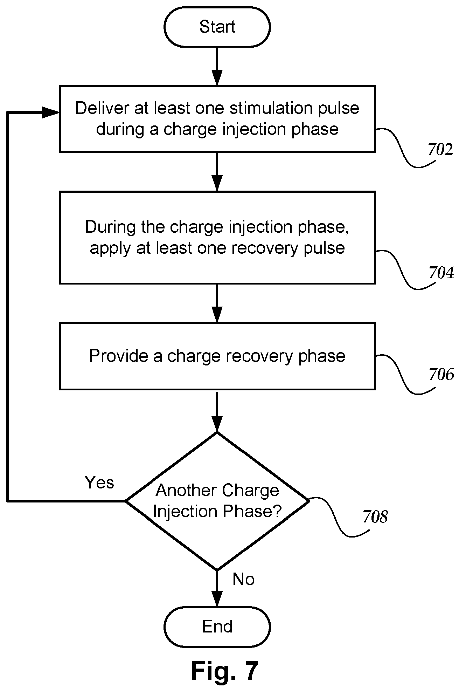

Figure 7 illustrates one method of delivering electrical stimulation. In step 702, at least one stimulation pulse is delivered through at least one stimulation electrode of at least one electrical stimulation lead to tissue of a patient during a charge injection phase.

In step 704, which occurs during 702, during the charge injection phase, at least one charge recovery pulse is applied. (It will be understood that, optionally, step 704 does not occur during every charge injection phase.) In at least some embodiments, the charge recovery pulse(s) are opposite in phase to the at least one stimulation pulse. Each of the charge recovery pulse(s) has a duration of no more than 30, 20, 10, 5, or 1 microseconds. In at least some embodiments, a charge injection pulse can be delivered between stimulation pulses. In at least some embodiments, a stimulation pulse can be delivered immediately prior to, and immediately after, the charge recovery pulse. In at least some embodiments, a charge recovery pulse can interrupt the delivery of a stimulation pulse and the stimulation pulse resumes after application of the charge recovery pulse.

In optional step 706, a charge recovery phase is provided after the charge injection phase. In at least some embodiments, one or more passive or active recovery phases may occur during the charge recovery phase.

In step 708, it is determined whether another charge injection phase is to be delivered. If so, the process returns to step 702. If not, the process ends.

It will be understood that each block of the flowchart illustration, and combinations of blocks in the flowchart illustration and methods disclosed herein, can be implemented by computer program instructions. These program instructions may be provided to a processor to produce a machine or engine, such that the instructions, which execute on the processor, create means for implementing the actions specified in the flowchart block or blocks or engine disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computing device. In addition, one or more processes may also be performed concurrently with other processes, or even in a different sequence than illustrated without departing from the scope or spirit of the invention.

The computer program instructions can be stored on any suitable computer-readable medium including, but not limited to, RAM, ROM, EEPROM, flash memory or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computing device. The computer program instructions can be stored locally or nonlocally (for example, in the Cloud).

The invention is defined in the appended claims. Aspects, embodiments and examples disclosed herein which do not fall within the scope of the appended claims do not form part of the invention and are merely provided for illustrative purposes. Furthermore, the methods presented in the present description are not claimed as such but are useful for understanding of the claimed subject matter, in particular since the methods can be performed by the claimed subject-matter.

## Claims

1. An electrical stimulation system (10, 400, 512), comprising:
at least one electrical stimulation lead (12), each of the at least one electrical stimulation lead comprising a plurality of stimulation electrodes (26); and
a processor (404, 502) coupled to the at least one electrical stimulation lead, wherein the processor executes instructions that perform actions, comprising:
directing delivery of at least one stimulation pulse (346, 346') through at least one of the stimulation electrodes of the at least one electrical stimulation lead to tissue of a patient during each of a plurality of charge injection phases (340), wherein each consecutive pair of the charge injection phases is separated by a charge recovery phase (342); and
for at least one of the at least one stimulation pulse:
during delivery of the stimulation pulse, directing application of at least one charge recovery pulse (344, 344') to interrupt the delivery of the stimulation pulse, wherein each one of the at least one charge recovery pulse has a relative amplitude that is larger in magnitude than an amplitude of the stimulation pulse; and
for each of the at least one charge recovery pulse, after application of the charge recovery pulse, directing resumption of delivery of the stimulation pulse at the amplitude of the stimulation pulse.

2. The electrical stimulation system of claim 1, wherein applying the at least one charge recovery pulses comprises applying a plurality of charge recovery pulses.

3. The electrical stimulation system of claim 2, wherein applying the plurality of charge recovery pulses comprises applying the plurality of charge recovery pulses periodically with a predetermined period.

4. The electrical stimulation system of claim 2, wherein applying the plurality of charge recovery pulses comprises successively applying each of the plurality of charge recovery pulses as an unrecovered charged delivered by the at least one stimulation pulse reaches or exceeds a predetermined threshold amount.

5. The electrical stimulation system of any one of claims 1 to 4, wherein the at least one charge recovery pulse recovers at least 10% of charge delivered by the at least one stimulation pulse of the at least one of the charge injection phases.

6. The electrical stimulation system of any one of claims 1 to 5, wherein the actions further comprise, during the charge recovery phase, provide at least one active recovery phase (248).

7. The electrical stimulation system of any one of claims 1 to 5, wherein the actions further comprise, during the charge recovery phase, provide at least one passive recovery phase (247).

8. The electrical stimulation system of any one of claims 1 to 7, further comprising a programming unit (408) configured to communicate with the processor to program the processor.

9. The electrical stimulation system of claim 8, wherein the programming unit is configured to receive user input or modification of a duration of the at least one charge recovery pulse and to communicate the user input to the processor.

10. The electrical stimulation system of claim 8, wherein the programming unit is configured to receive user input or modification of an amplitude or a relative amplitude of the at least one charge recovery pulse and to communicate the user input to the processor.

11. An electrical stimulation system (10, 400, 512), comprising:
at least one electrical stimulation lead (12), each of the at least one electrical stimulation lead comprising a plurality of stimulation electrodes (26); and
a processor (404, 502) coupled to the at least one electrical stimulation lead, wherein the processor executes instructions that perform actions, comprising:
directing delivery of at least one stimulation pulse (346, 346') through at least one of the stimulation electrodes of the at least one electrical stimulation lead to tissue of a patient during each of a plurality of charge injection phases (340), wherein each consecutive pair of the charge injection phases is separated by a charge recovery phase (342); and
during at least one of the charge injection phases, applying at least one charge recovery pulse (344, 344') to interrupt the delivery of the at least one stimulation pulse of the at least one of the charge injection phases, wherein each of the at least one charge recovery pulse has a duration of no more than 20 microseconds.

12. The electrical stimulation system of claim 11, wherein the directing comprises directing delivery of a one of the at least one stimulation pulse immediately prior to, and immediately after, the application of a one of the least one charge recovery pulse.

13. The electrical stimulation system of any one of claims 11 or 12, wherein the applying comprises interrupting the delivery of a one of the least one stimulation pulse by the application of a one of the at least one charge recovery pulse, and
wherein the directing delivery comprises resuming the one of the at least one stimulation pulse following the application of the one of the at least one charge recovery pulse.

14. The electrical stimulation system of any one of claims 11 to 13, wherein the at least one charge recovery pulse recovers at least 10% of charge delivered by the at least one stimulation pulse of the at least one of the charge injection phases.

15. The electrical stimulation system of any one of claims 11 to 14, wherein the actions further comprise, during the charge recovery phase, provide at least one active recovery phase or at least one passive recovery phase.

## Patentansprüche

1. Elektrisches Stimulationssystem (10, 400, 512), aufweisend:
mindestens eine elektrische Stimulationsleitung (12), wobei jede der mindestens einen elektrischen Stimulationsleitung eine Vielzahl von Stimulationselektroden (26) aufweist; und
einen Prozessor (404, 502), der mit der mindestens einen elektrischen Stimulationsleitung verbunden ist, wobei der Prozessor Befehle ausführt, die Aktionen ausführen, aufweisend:
Anweisen der Abgabe mindestens eines Stimulationsimpulses (346, 346') durch mindestens eine der Stimulationselektroden der mindestens einen elektrischen Stimulationsleitung an Gewebe eines Patienten während jeder einer Vielzahl von Ladungsinjektionsphasen (340), wobei jedes aufeinanderfolgende Paar der Ladungsinjektionsphasen durch eine Ladungsrückgewinnungsphase (342) getrennt ist; und
für mindestens einen des mindestens einen Stimulationsimpulses:
während der Abgabe des Stimulationsimpulses: Anweisen der Bereitstellung mindestens eines Ladungsrückgewinnungsimpulses (344, 344') zum Unterbrechen der Abgabe des Stimulationsimpulses, wobei jeder unter dem mindestens einen Ladungsrückgewinnungsimpuls eine relative Amplitude aufweist, die größer ist als die Amplitude des Stimulationsimpulses; und
für jeden unter dem mindestens einen Ladungsrückgewinnungsimpuls, nach dem Zuführen des Ladungsrückgewinnungsimpulses: Anweisen der Wiederaufnahme der Abgabe des Stimulationsimpulses mit der Amplitude des Stimulationsimpulses.

2. Elektrisches Stimulationssystem nach Anspruch 1, wobei das Bereitstellen des mindestens einen Ladungsrückgewinnungsimpulses das Bereitstellen einer Vielzahl von Ladungsrückgewinnungsimpulsen aufweist.

3. Elektrisches Stimulationssystem nach Anspruch 2, wobei das Bereitstellen der Vielzahl von Ladungsrückgewinnungsimpulsen das periodische Bereitstellen der Vielzahl von Ladungsrückgewinnungsimpulsen mit einer vorgegebenen Periode aufweist.

4. Elektrisches Stimulationssystem nach Anspruch 2, wobei das Bereitstellen der Vielzahl von Ladungsrückgewinnungsimpulsen das aufeinanderfolgende Bereitstellen jedes der Vielzahl von Ladungsrückgewinnungsimpulsen aufweist, wenn eine nicht zurückgewonnene Ladung, die durch den mindestens einen Stimulationsimpuls abgegeben wird, einen vorgegebenen Schwellenwert erreicht oder überschreitet.

5. Elektrisches Stimulationssystem nach einem der Ansprüche 1 bis 4, wobei der mindestens eine Ladungsrückgewinnungsimpuls mindestens 10% der Ladung zurückgewinnt, die durch den mindestens einen Stimulationsimpuls der mindestens einen der Ladungsinjektionsphasen abgegeben wurde.

6. Elektrisches Stimulationssystem nach einem der Ansprüche 1 bis 5, wobei die Aktionen ferner aufweisen: Bereitstellen mindestens einer aktiven Rückgewinnungsphase (248) während der Ladungsrückgewinnungsphase.

7. Elektrisches Stimulationssystem nach einem der Ansprüche 1 bis 5, wobei die Aktionen ferner aufweisen: Bereitstellen mindestens einer passiven Rückgewinnungsphase (247)während der Ladungsrückgewinnungsphase.

8. Elektrisches Stimulationssystem nach einem der Ansprüche 1 bis 7, ferner aufweisend eine Programmiereinheit (408), die dafür konfiguriert ist, mit dem Prozessor zu kommunizieren, um den Prozessor zu programmieren.

9. Elektrisches Stimulationssystem nach Anspruch 8, wobei die Programmiereinheit dafür konfiguriert ist, Benutzereingaben oder eine Änderung einer Dauer des mindestens einen Ladungsrückgewinnungspulses zu empfangen und die Benutzereingaben an den Prozessor zu übermitteln.

10. Elektrisches Stimulationssystem nach Anspruch 8, wobei die Programmiereinheit dafür konfiguriert ist, Benutzereingaben oder eine Änderung einer Amplitude oder einer relativen Amplitude des mindestens einen Ladungsrückgewinnungspulses zu empfangen und die Benutzereingaben an den Prozessor zu übermitteln.

11. Elektrisches Stimulationssystem (10, 400, 512), aufweisend:
mindestens eine elektrische Stimulationsleitung (12), wobei jede der mindestens einen elektrischen Stimulationsleitung eine Vielzahl von Stimulationselektroden (26) aufweist; und
einen Prozessor (404, 502), der mit der mindestens einen elektrischen Stimulationsleitung verbunden ist, wobei der Prozessor Befehle ausführt, durch die Aktionen ausgeführt werden, aufweisend:
Anweisen der Abgabe mindestens eines Stimulationsimpulses (346, 346') durch mindestens eine der Stimulationselektroden der mindestens einen elektrischen Stimulationsleitung an Gewebe eines Patienten während jeder einer Vielzahl von Ladungsinjektionsphasen (340), wobei jedes aufeinanderfolgende Paar der Ladungsinjektionsphasen durch eine Ladungsrückgewinnungsphase (342) getrennt ist; und
während mindestens einer der Ladungsinjektionsphasen: Bereitstellen mindestens eines Ladungsrückgewinnungsimpulses (344, 344') zum Unterbrechen der Abgabe des mindestens einen Stimulationsimpulses der mindestens einen der Ladungsinjektionsphasen, wobei jeder der mindestens einen Ladungsrückgewinnungsimpulse eine Dauer von nicht mehr als 20 Mikrosekunden hat.

12. Elektrisches Stimulationssystem nach Anspruch 11, wobei das Anweisen das Anweisen der Abgabe eines des mindestens einen Stimulationsimpulses unmittelbar vor und unmittelbar nach dem Bereitstellen eines des mindestens einen Ladungsrückgewinnungsimpulses aufweist.

13. Elektrisches Stimulationssystem nach Anspruch 11 oder 12, wobei das Bereitstellen das Unterbrechen der Abgabe eines des mindestens einen Stimulationsimpulses durch das Bereitstellen eines des mindestens einen Ladungsrückgewinnungsimpulses aufweist, und
wobei das Anweisen der Abgabe das Zurückgewinnen des einen des mindestens einen Stimulationsimpulses nach dem Bereitstellen des einen des mindestens einen Ladungsrückgewinnungsimpulses aufweist.

14. Elektrisches Stimulationssystem nach einem der Ansprüche 11 bis 13, wobei der mindestens eine Ladungsrückgewinnungsimpuls mindestens 10% der Ladung zurückgewinnt, die durch den mindestens einen Stimulationsimpuls der mindestens einen der Ladungsinjektionsphasen abgegeben wurde.

15. Elektrisches Stimulationssystem nach einem der Ansprüche 11 bis 14, wobei die Aktionen ferner aufweisen: Bereitstellen mindestens einer aktiven Rückgewinnungsphase oder mindestens einer passiven Rückgewinnungsphase während der Ladungsrückgewinnungsphase.

## Revendications

1. Système de stimulation électrique (10, 400, 512), comprenant :
au moins un fil de stimulation électrique (12), chacun des au moins un fil de stimulation électrique comprenant une pluralité d'électrodes de stimulation (26) ; et
un processeur (404, 502) couplé avec le au moins un fil de stimulation électrique, dans lequel le processeur exécute des instructions qui réalisent des actions, comprenant :
l'instruction de la fourniture d'au moins une impulsion de stimulation (346, 346') à travers au moins l'une des électrodes de stimulation du au moins un fil de stimulation électrique vers un tissu d'un patient pendant chacune parmi une pluralité de phases d'injection de charge (340), dans lequel chaque pair consécutive des phases d'injection de charge est séparée par une phase de récupération de charge (342) ; et
pour au moins l'une des au moins une impulsion de stimulation :
pendant la fourniture de l'impulsion de stimulation, l'instruction de l'application d'au moins une impulsion de récupération de charge (344, 344') pour interrompre la fourniture de l'impulsion de stimulation, dans lequel chacune de la au moins une impulsion de récupération de charge présente une amplitude relative qui est supérieure par l'ampleur à une amplitude de l'impulsion de stimulation ; et
pour chacune de la au moins une impulsion de récupération de charge, après application de l'impulsion de récupération de charge, l'instruction de la reprise de fourniture de l'impulsion de stimulation avec l'amplitude de l'impulsion de stimulation.

2. Système de stimulation électrique selon la revendication 1, dans lequel l'application des au moins une impulsion de récupération de charge comprend l'application d'une pluralité d'impulsions de récupération de charge.

3. Système de stimulation électrique selon la revendication 2, dans lequel l'application de la pluralité d'impulsions de récupération de charge comprend l'application périodique de la pluralité d'impulsions de récupération de charge avec une période prédéterminée.

4. Système de stimulation électrique selon la revendication 2, dans lequel l'application de la pluralité d'impulsions de récupération de charge comprend l'application successive de chacune parmi la pluralité d'impulsions de récupération de charge tandis qu'une charge non récupérée fournie par la au moins une impulsion de stimulation atteint ou dépasse un niveau de seuil prédéterminé.

5. Système de stimulation électrique selon l'une quelconque des revendications 1 à 4, dans lequel la au moins une impulsion de récupération de charge récupère au moins 10 % d'une charge fournie par la au moins une impulsion de stimulation de la au moins une des phases d'injection de charge.

6. Système de stimulation électrique selon l'une quelconque des revendications 1 à 5, dans lequel les actions comprennent en outre, pendant la phase de récupération de charge, la fourniture d'au moins une phase de récupération active (248).

7. Système de stimulation électrique selon l'une quelconque des revendications 1 à 5, dans lequel les actions comprennent en outre, pendant la phase de récupération de charge, la fourniture d'au moins une phase de récupération passive (247).

8. Système de stimulation électrique selon l'une quelconque des revendications 1 à 7, comprenant en outre une unité de programmation (408) configurée pour communiquer avec le processeur afin de programmer le processeur.

9. Système de stimulation électrique selon la revendication 8, dans lequel l'unité de programmation est configurée pour recevoir une entrée d'utilisateur ou une modification d'une durée de la au moins une impulsion de récupération de charge et pour communiquer l'entrée d'utilisateur au processeur.

10. Système de stimulation électrique selon la revendication 8, dans lequel l'unité de programmation est configurée pour recevoir une entrée d'utilisateur ou une modification d'une amplitude ou d'une amplitude relative de la au moins une impulsion de récupération de charge et pour communiquer l'entrée d'utilisateur au processeur.

11. Système de stimulation électrique (10, 400, 512), comprenant :
au moins un fil de stimulation électrique (12), chacun des au moins un fil de stimulation électrique comprenant une pluralité d'électrodes de stimulation (26) ; et
un processeur (404, 502) couplé avec le au moins un fil de stimulation électrique, dans lequel le processeur exécute des instructions qui réalisent des actions, comprenant :
l'instruction de la fourniture d'au moins une impulsion de stimulation (346, 346') à travers au moins l'une des électrodes de stimulation du au moins un fil de stimulation électrique vers un tissu d'un patient pendant chacune parmi une pluralité de phases d'injection de charge (340), dans lequel chaque pair consécutive des phases d'injection de charge est séparée par une phase de récupération de charge (342) ; et
pendant au moins l'une des phases d'injection de charge, l'application d'au moins une impulsion de récupération de charge (344, 344') pour interrompre la fourniture de la au moins une impulsion de stimulation de la au moins une des phases d'injection de charge, dans lequel chacune de la au moins une impulsion de récupération de charge présente une durée de tout au plus 20 microsecondes.

12. Système de stimulation électrique selon la revendication 11, dans lequel l'instruction comprend l'instruction de la fourniture d'une de la au moins une impulsion de stimulation immédiatement antérieurement, et immédiatement après, l'application d'une de la au moins une impulsion de récupération de charge.

13. Système de stimulation électrique selon l'une quelconque des revendications 11 ou 12, dans lequel l'application comprend l'interruption de la fourniture d'une de la au moins une impulsion de stimulation par l'application d'une de la au moins une impulsion de récupération de charge, et
dans lequel l'instruction de la fourniture comprend la reprise de de l'une de la au moins une impulsion de stimulation à la suite de l'application de l'une de la au moins une impulsion de récupération de charge.

14. Système de stimulation électrique selon l'une quelconque des revendications 11 à 13, dans lequel la au moins une impulsion de récupération de charge récupère au moins 10 % d'une charge fournie par la au moins une impulsion de stimulation de la au moins une des phases d'injection de charge.

15. Système de stimulation électrique selon l'une quelconque des revendications 11 à 14, dans lequel les actions comprennent en outre, pendant la phase de récupération de charge, la fourniture d'au moins une phase de récupération active ou d'au moins une phase de récupération passive.
